(19) Europäisches Patentamt
European Patent Office
Office européen des brevets

(11) **EP 1 617 853 B1**

## (12) EUROPEAN PATENT SPECIFICATION

(45) Date of publication and mention
of the grant of the patent:
**24.10.2007 Bulletin 2007/43**

(51) Int Cl.:
***A61K 36/185*** *(2006.01)*        ***A61K 31/7034*** *(2006.01)*
***A61P 1/16*** *(2006.01)*

(21) Application number: **03816508.0**

(22) Date of filing: **31.03.2003**

(86) International application number:
**PCT/IB2003/001180**

(87) International publication number:
**WO 2004/087184 (14.10.2004 Gazette 2004/42)**

(54) **ISOLATION OF THE HEPATOPROTECTIVE AGENT ACTEOSIDE FROM COLERBROKEA OPPOSITIFOLIA**

ISOLIERUNG DES HEPATOPROTEKTIVEN WIRKSTOFFES ACTEOSID AUS COLERBROOKEA OPPOSITIFOLIA

ISOLEMENT DE L'AGENT HEPATOPROTECTEUR ACTEOSIDE DE LA PLANTE COLERBRO0KEA OPPOSITIFOLIA

(84) Designated Contracting States:
**AT BE BG CH CY CZ DE DK EE ES FI FR GB GR HU IE IT LI LU MC NL PT RO SE SI SK TR**

(43) Date of publication of application:
**25.01.2006 Bulletin 2006/04**

(73) Proprietor: **COUNCIL OF SCIENTIFIC AND INDUSTRIAL RESEARCH**
**New Delhi 110 001 (IN)**

(72) Inventors:
• **QAZI, Ghulam, Nabi**
**180 001 Jammu (IN)**
• **SURI, Om, Parkash**
**180 001 Jammu (IN)**
• **BEDI, Kasturi, Lal**
**180 001 Jammu (IN)**
• **SURI, Krishan, Avtar**
**180 001 Jammu (IN)**
• **GUPTA, Bishan, Datta**
**180 001 Jammu (IN)**
• **JAGGI, Bupinder, Singh**
**180 001 Jammu (IN)**
• **KAPAHI, Bal, Krishan**
**180 001 Jammu (IN)**
• **SATTI, Naresh, Kumar**
**180 001 Jammu (IN)**
• **AMINA, Musarat**
**180 001 Jammu (IN)**
• **CHANDAN, Bal, Krishan**
**180 001 Jammu (IN)**
• **SHARMA, Neelam**
**180 001 Jammu (IN)**
• **SINGH, Gurdarshan**
**180 001 Jammu (IN)**

(74) Representative: **van Westenbrugge, Andries**
**Nederlandsch Octrooibureau**
**Postbus 29720**
**2502 LS Den Haag (NL)**

(56) References cited:
**US-A- 5 719 129**

• **XIONG QUANBO ET AL: "Hepatoprotective activity of phenylethanoids from Cistanche deserticola." PLANTA MEDICA, vol. 64, no. 2, March 1998 (1998-03), pages 120-125, XP009012741 ISSN: 0032-0943 cited in the application**
• **DONG SOOL YIM ET AL: "Biological activities of verbascoside from Pedicularis resupinata var. oppositifolia" KOREAN JOURNAL OF PHARMACOGNOSY 1997 SOUTH KOREA, vol. 28, no. 4, 1997, pages 252-256, XP009012737 ISSN: 0253-3073**
• **XIONG QUANBO ET AL: "Acteoside inhibits apoptosis in D-Galactosamine and lipopolysaccharide-induced liver injury." LIFE SCIENCES, vol. 65, no. 4, 18 June 1999 (1999-06-18), pages 421-430, XP001147700 ISSN: 0024-3205**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

• HOUGHTON P J ET AL: "Anti-hepatotoxic activity of extracts and constituents of Buddleja species" PLANTA MEDICA 1989 GERMANY, vol. 55, no. 2, 1989, pages 123-126, XP009012740 ISSN: 0032-0943 cited in the application

**Description**

**Field of the present invention**

A process of isolation of pure Acteoside of high hepatoprotection from plant *Colerbrookea oppositifolia,* said process comprising steps of drying aerial parts of the plant, grinding the dried parts into powder, percolating the powder with water or ethanol for 3-4 times to obtain an extract, filtering the extract for clearing suspended particles to obtain supernatant, drying the supernatant at about 45 to 55°C to obtain a residue, fractionating the residue with chloroform, ethyl acetate, and butanol successively, subjecting butanol fraction to adsorption chromatography of $SiO_2$ after adding methanol to the fraction, charging the adsorbed fraction to glass column, eluting the column with solvents of increasing polarity of methanol : chloroform to obtain further fractions and repeating the process one more time, subjecting the fractions to column chromatography to obtain fractions, concentrating the fractions under reduced pressure to obtain acteoside as residue; also disclosed is a method of effectively hepatoprotecting a subject using pure Acteoside from plant *Colerbrookea oppositifolia,* said method comprising steps of administering appropriate low-dose of the acteoside to the subject.

**Background of the present invention**

Phenylethanoids are a class of compounds reported to be present in plants like *Cistanche deserticola* and *Buddleja species* (Quanbo Xing, Koji Hase et al, Planta Medica, 64, 120-125 (1998); Peter J. Houghton and Hiroshi Hikino. Planta Medica, Vol .55, 123-126 (1989).

It is for the first time that a phenylethanoid - verbascoside (also called acteoside or kusaginin) has been isolated by the authors from *Colebrookea oppositifolia* and found to possess a very strong antihepatotoxic/ hepatoprotective activity at unusually very low doses (between 1.25 and 2.5 mg /kg) in rats and mice.

The presence of number of flavonoids and glycoflavonoids has already been reported in the literature from *Colebrookea oppositifolia* [S. Aziz Ahmed, S.A. Siddiqui and Asif Zaman, Indian J. Chemistry 12 1327-28 (1974); S.A. Patwardhan and A.S.Gupta, Indian J. Chemistry, 20B, 627, (1981); Fan Yank, Xing-Li, HAN-Qing Wang and Chong-Ren Yang, Phytochemistry 42 , 867-69 (1996)]. However, the presence of Acteoside from this plant is being reported for the first time.

Antifertility activity of this plant in male rats with special reference to testicular cell population dynamics have earlier been reported (R.S. Gupta, R.J. Yadav, V.P. Dixit and M.P. Dobhal, Fitoterapia, 72, 236-45 (2001).

Though the Hepatoprotective/Antihepatotoxic activity of Acteoside (Verbascoside) isolated from other two plants viz. *Cistanche* species and *Buddleja* species has been reported. (Quanbo Xing, Koji Hase et al, Planta Medica, 64, 120-125 (1998); Peter J. Houghton and Hiroshi Hikino. Planta Medica, Vol .55, 123-126 (1959), however the present invention not only reports a distinct source namely *Colebrookea oppositifolia* but the desired activity is accomplished at unusually very low dosage between 1.5 to 2.5 mg/kg in rats.

As already stated above the Hepatoprotective/Antihepatotoxic activity of acteoside has been reported both *in vitro* and *in vivo* (Quanbo Xing, Koji Hase et al, Planta Medica, 64, 120-125 (1998). But the present invention describes the isolation of acteoside from a hitherto unreported source in such a way that the compound shows antihepatotoxic/ hepatoprotective activity at doses nearly 12 to 25 times lower than those already reported in the prior art (Quanbo Xing, Koji Hase et al, Planta Medica, 64, 120-125 (1998); Peter J. Houghton and Hiroshi Hikino. Planta Medica, Vol .55, 123-126 (1989). Moreover the parameters evaluated in the prior reports are limited to ALT (*in vivo*) and AST & MDA (*in vitro*) whereas the present invention describes the influence of acteoside practically in all important parameters leading to for a clear decision on the efficacy of any hepatoprotective or antihepatotoxic product.

The reason for the activity of acteoside at such low doses compared to the one reported in the prior art may be due to the specific method of its isolation being distinct to yield a product of higher purity. Contaminants in phytochemicals are well documented to seriously jeopardize the activity of pure active compound though there could be synergistic action also of which, however, there in no mention or indication in the prior art.

**Objects of the present invention**

The main object of the present invention is to isolate a hepatoprotective from plant *Colerbrookea oppositifolia.*

Another object of the present invention is to develop a process for the isolation of acteoside from plant *Colerbrookea oppositifolia.*

Yet another object of the present invention is to develop a method of hepatoprotecting a subject using *Colerbrookea oppositifolia.*

## Summary of the present invention

A process of isolation of pure Acteoside of high hepatoprotection from plant *Colerbrookea oppositifolia* said process comprising steps of drying aerial parts of the plant grinding the dried parts into powder, percolating the powder with water or ethanol for 3-4 times to obtain an extract, filtering the extract for clearing suspended particles to obtain supernatant, drying the supernatant at about 45 to 55°C to obtain a residue, fractionating the residue with chloroform, ethyl acetate, and butanol successively, subjecting butanol fraction to adsorption chromatography of $SiO_2$ after adding methanol to the fraction, charging the adsorbed fraction to glass column, eluting the column with solvents of increasing polarity of methanol : chloroform to obtain further fractions and repeating the process one more time, subjecting the fractions to column chromatography to obtain fractions, concentrating the fractions under reduced pressure to obtain acteoside as residue; and hepatoprotecting a subject using pure Acteoside from plant *Colerbrookea oppositifolia* comprising steps of administering appropriate low-dose of the acteoside to the subject.

## Detailed description of the present invention

A process of isolation of pure Acteoside of high hepatoprotection from plant *Colerbrookea oppositifolia,* said process comprising steps of drying aerial parts of the plant, grinding the dried parts into powder, percolating the powder with water or ethanol for 3-4 times to obtain an extract, filtering the extract for clearing suspended particles to obtain supernatant, drying the supernatant at about 45 to 55°C to obtain a residue, fractionating the residue with chloroform, ethyl acetate, and butanol successively, subjecting butanol fraction to adsorption chromatography of $SiO_2$ after adding methanol to the fraction, charging the adsorbed fraction to glass column, eluting the column with solvents of increasing polarity of methanol : chloroform to obtain further fractions and repeating the process one more time, subjecting the fractions to column chromatography to obtain fractions, concentrating the fractions under reduced pressure to obtain acteoside as residue; and hepatoprotecting a subject using pure Acteoside from plant *Colerbrookea oppositifolia* comprising steps of administering appropriate low-dose of the acteoside to the subject.

In an embodiment of the present invention, wherein a process of isolation of pure Acteoside of high hepatoprotection from plant *Colerbrookea oppositifolia,* said process comprising steps of:

- drying aerial parts of the plant,
- grinding the dried parts into powder,
- percolating the powder with water or ethanol for 3-4 times to obtain an extract,
- filtering the extract for clearing suspended particles to obtain supernatant,
- drying the supernatant at about 45 to 55°C to obtain a residue,
- fractionating the residue with chloroform, ethyl acetate, and butanol successively,
- subjecting butanol fraction to adsorption chromatography of $SiO_2$ after adding methanol to the fraction,
- charging the adsorbed fraction to glass column,
- eluting the column with solvents of increasing polarity of methanol : chloroform to obtain further fractions and repeating the process one more time,
- subjecting the fractions to column chromatography to obtain fractions,
- concentrating the fractions under reduced pressure to obtain acteoside as residue.

In another embodiment of the present invention, wherein the acteoside obtained by the process shows hepatoprotective activity at 12 to 25 times lesser dosage as compared to the one obtained from other sources.

In yet another embodiment of the present invention, wherein the extracts are aqueous and alcoholic extracts.

In still another embodiment of the present invention, wherein acteoside is about 1.0% (wt.) of the total extract.

Also disclosed is a method of effectively hepatoprotecting a subject using pure Acteoside from plant *Colerbrookea oppositifolia,* said method comprising steps of administering appropriate low-dose of the acteoside to the subject.

The dosage may range between 0.5 to 10.0 mg/kg body weight.

The acteoside may be administered through P.O. routes.

The acteoside may reduce the abnormally elevated levels of serum glutamine transferase (GPT).

The acteoside may reduce the abnormally elevated levels of serum glutamine transferase (GOT).

The acteoside may reduce the abnormally elevated levels of serum alkaline phosphatase (ALP).

The acteoside may reduce the abnormally elevated levels of serum Bilirubin.

The acteoside may reduce the abnormally elevated levels of serum triglycerides (TG).

The acteoside may reduce the abnormally elevated levels of lipid peroxidase (LP).

The acteoside may increase the abnormally reduced levels of albumin.

The acteoside may increase the abnormally decreased levels of reduced-glutathione.

The acteoside may be about 10 to 20 times more effective as compared to commercially available hepatoprotectants.

The acteoside may provide about 40-85% protection against hepatotoxicity.

The present invention relates to unusually very low dosage antihepatotoxic / hepatoprotective activity, of a phenylethanoid named as Verbascoside-also termed as Acteoside or Kusaginin isolated and reported for the first time from a plant named *Colerbrookea oppositifolia.* The invention describes the isolation of verbascoside in a specific manner and demonstrates antihepatotoxic / hepatoprotective activity at very low doses against different toxins.

The active compound acteoside (verbascoside) is being reported for the first time by the authors of invention from the plant *Colebrookea oppositifolia.* The authenticity of the acteoside isolated by the authors has been confirmed by $^1$H and $^{13}$C NMR data similar to reported in literature (Phytochemistry 21, 1123-1127 (1982).

### Brief description of the accompanying drawings

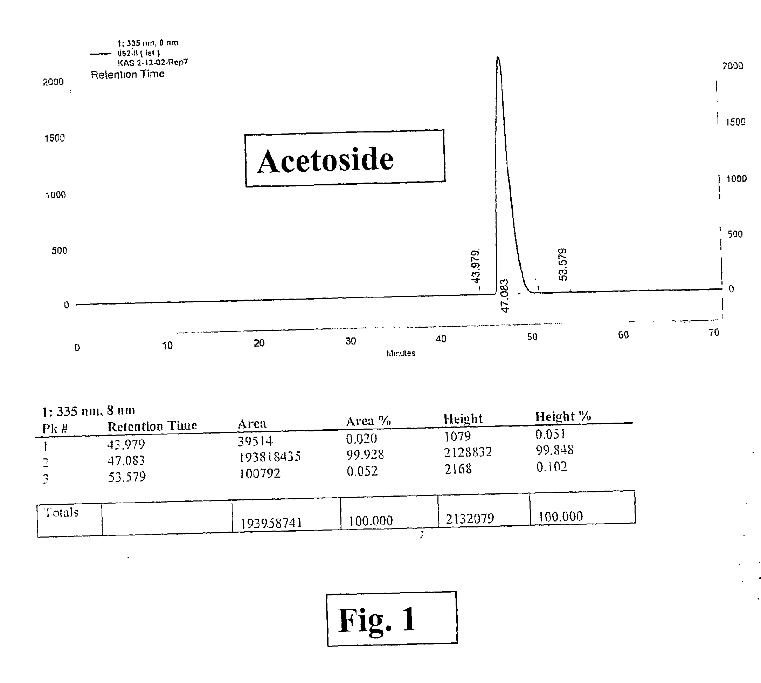

**Fig 1** shows HPLC graph confirming purity.

**Fig 2** shows comparative hepatoprotective activity (%) of acteoside, silymarin, and glycyrrhizia.

The process of isolation of acteoside from different extracts (95% alc., 50% alc. and aqueous) of the plant is described in the examples appended herewith as Annexure-1. The HPLC graph confirming purity is enclosed as fig-1.

The compound has been bioevaluated for hepatoprotective/antihepatotoxic activity by using a comprehensive study design annexed herewith as Annexure-2. The parameters studied during the bioevaluation to establish the bioefficacy and optimum dose were preferred using the kits (Clonital, Italy and Accurex Biomedical Pvt. Ltd Thane). The list of the parameters is given in Annexure-2 appended herewith. The dose response graphs with respect to different parameters in comparison with the standard drug with silymarin and glycyrrhizin are given in Annexure-3 appended herewith.

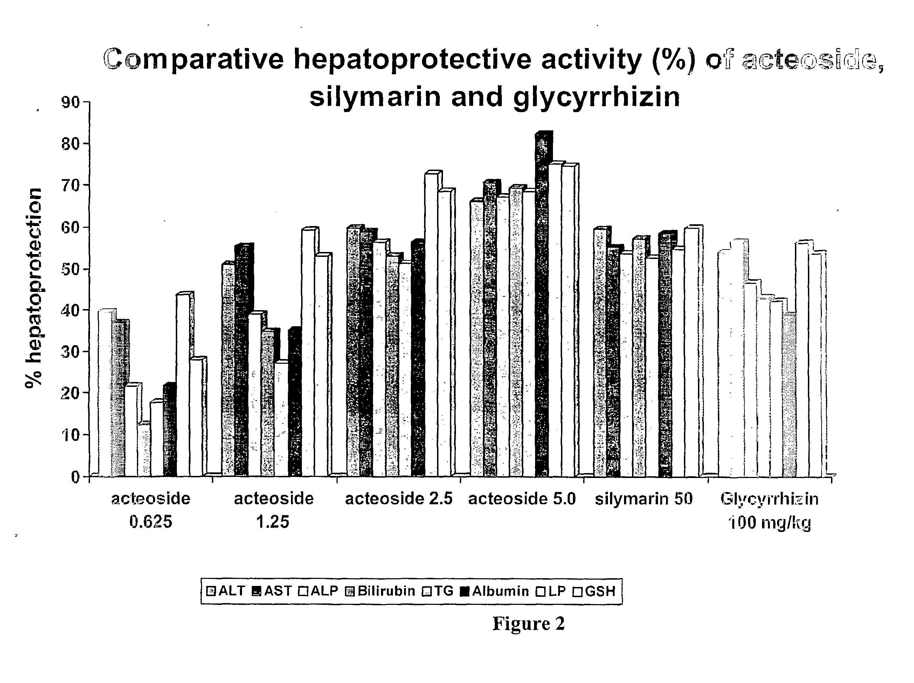

The bioevaluation data for each dose of the acteoside as compared to reference materials viz. silymarin, glycyrrhizin, and required controls are given in the Table-1 which is appended herewith the invention. As is clear from the figure 2 and Table-1 all the parameters which were compromised after the administration of hepatotoxin $CCl_4$ have a very good tendency to revert back to normal levels from a dose of 1.25 mg upto 5 mg/kg of acteoside. The overall percent protection provided by acteoside with reference to all the parameters studied in the invention is generally from 40-80%, and is better than that rendered by silymarin even at 10-20 times the dosage of acteoside.

The optimum dose to achieve this protection with acteoside of the invention lies between 1.25 mg/kg and 2.5 mg/kg. Statistically, there is no significance observed between the three doses viz. 1.25, 2.5 and 5 mg/kg except when 1.25 mg/kg dose data is compared with that of 5mg/kg dose (Table-2). But no statistical significance is observable between higher doses i.e. 2.5mg/kg and 5 mg/kg. At the same time, similar observation holds fully well for dose effect between 1.25 and 2.5 mg/kg.

The above-stated invention is elaborated in the form of examples and should not be construed to limit the scope of the invention

### Example 1:

Dried aerial parts of plant material *Colebrookea oppositifolia* (500 gm) were ground to a coarse powder. The powder was percolated with 95% ethanol for fourteen hours. Extraction process was repeated four times using total of 9 litres of 95% ethanol (3.0 +2.0 + 2.0 + 2.0 Litre, four extractions). All the four extracts were pooled and filtered clear of suspended particles. The supernatant was evaporated to dryness on a wiped film evaporator at $50\pm5°C$. Residue obtained was 60.0 g, (coded as RJM/0862/P0S/A001) extractive value 12%.

The extract was fractionated with $CHCl_3$, EtOAc and *n*-BuOH (2 x1 Litre each) successively. 15.0 g *n*-BuOH extract was subjected to adsorption chromatography after dissolving in minimum quantity of MeOH, and adsorbing on $SiO_2$ gel, 100-200 mesh (100 gm.) Solvent was completely removed to get free flowing material. The adsorbed extract was charged in a glass column of 37.5 mm Ø. The column was eluted with solvents by gradually increasing the %age of MeOH in $CHCl_3$, 105 fractions of 100 ml each were collected and pooled on the bases of TLC patterns checked by using EtOAc: HCOOH : $H_2O$ : : 8:1:1 as mobile phase. Spots were visualised by spraying with freshly prepared Borinate-PEG 4000 solution [1% solution of 2-aminoethyldiphenylborinate in MeOH and 5% solution of polyethylene glycol 4000 in EtOH (mixed 1:1 v/v before spraying)].

Seven of the fractions showing same TLC pattern were pooled, dried and subjected to rechromatography using 100-200 mesh $SiO_2$ gel column (1:20 ratio) and eluted with $CHCl_3$ : MeOH mixtures of increasing polarity. In all 60 fractions of 200 ml each were collected. Eight of the fractions were pooled on the bases of TLC were dried and again subjected to column chromatography. 30 fractions of 100 ml each were collected. Six fractions were concentrated under reduced pressure. Residue was crystallised from MeOH / CHCl$_3$ as a colourless amorphous powder, soluble in MeOH . Compound found at Rf 0.42 , (solvent system EtOAc: HCOOH : H$_2$O: : 8:1:1) was coded as 862-II. The purity of 862-II was established on the basis of HPLC using following operating conditions.

| | |
|---|---|
| Column : | RP-18e (E-Merck, 5$\mu$m, 4.6 $\times$ 250 mm), at |
| Mobile Phase : | Acetonitrile (B) : 1.5% acetic acid in water (A) |
| Flow rate : | 1ml / min. |
| $\lambda$max : | 335 nm |

The compound was established as Verbascoside on the basis of NMR ($^1$H and $^{13}$C) and FABMS data.

**Example 2**

Dried aerial parts (500 gm) of *Colebrookea oppositifolia* were ground and percolated with 50% aqueous ethanol four times (50% EtOH, 4 x 2.5 Litre) for 14 hrs each. All the four extracts were pooled. The pooled aqueous extracts were centrifuged, clear supernatant was evaporated to dryness on a wiped film evaporator at 50 $\pm$ 5°C. The residue (90 g) was coded as RJM/0862/P08/A002 (extractive value 18%) and fractionated with CHCl$_3$, EtOAc and *n*-BuOH successively.

The *n*-BuOH ext. was chromatographed on a column of silica gel (60-120 mesh) eluted with a gradient of MeOH in CHCl$_3$. The CHCl$_3$ : T MeOH (5:1) eluate was rechromatographed on a silica gel (100-200 mesh) column using CHCl$_3$-MeOH : H$_2$O (6:1:0.1) as solvent. Fractions homogeneous on TLC were pooled, dried and charged on a sephadex LH-20 column, eluted with MeOH to produce two fractions of 500 ml each. Second fraction containing mainly the target compound (862-n) was subjected to further purification over a sephadex. LH-20. Column was eluted with MeOH: H$_2$O (3:2) to afford a fraction, which on crystallisation from MeOH/CHCl$_3$ yielded a colourless amorphous powder soluble in McOH , Rf0.42 (solvent system EtOAc:HCOOH : H$_2$O:: S:1:1) .

Standardisation of the extract RJM/OS62/POS/A002 was carried out on the basis of S62-II by HPLC using following operating conditions:

| | |
|---|---|
| Column : | RP- 1 8e (E-Merck, 5$\mu$m, 4.6$\times$250 mm), at |
| Mobile Phase : | Acetonitrile (B) : 1.5% acetic acid in water (A) |
| Flow rate : | 1ml/min. |
| $\lambda$max : | 335 nm |

The compound was established as Verbascoside on the basis of NMR ($^1$H and $^{13}$C) and FABMS data.

The %age of 862-II in the extract RJM/0862/P08/A002 was found to be 0.86.

**Example 3**

*Colebrookea oppositifolia* aerial parts (500 g) were ground to a coarse powder and then extracted with deionised water at 98$\pm$ 1°C for 2 hrs. Extraction process was repeated four times using total water (1 + 3 x 0.7 Litre, four extractions). All the four extracts were pooled The pooled extract was centrifuged, the clear filtrate was lyophilized to get light yellow amorphous powder (yield 95 gm). Aqueous extract residue and coded as RJM/0862/P08/A003 (extractive value 19%) was fractionated with CHCl$_3$ , EtOAc and *n*-BuOH successively.

*n*-BuOH extract was subjected to adsorption chromatography on SiO$_2$ gel, 60-120 mesh (150 gm). Solvent was completely removed to get free flowing material. A glass column of 1.5 inch dia was packed with 100 gm SiO$_2$ gel, 60-120 mesh in EtOAc. The adsorbed material was charged in the column. The column was eluted with EtOAc and by gradually increasing the %age with MeOH. In all 120 fractions of 100 ml each were collected and pooled on the basis of TLC pattern (EtOAc: HCOOH : H$_2$O :: 8:1:1 as developing solvent). Spots were visualised by spraying with freshly prepared Borinate-PEG 4000 solution [1% solution of 2-aminoethyldiphenylborinate in MeOH and 5% solution of polyethylene glycol 4000 in EtOH (mixed 1:1 v/v before spraying)].

Fractions eluted in EtOAc and 10% MeOH showed same TLC pattern. These fractions were pooled, dried and then dissolved in minimum quantity of MeOH . Crystallisation was carried out by the addition of CHCl$_3$ in small portions to methanol solution which yielded a colourless amorphous powder characterised as 862-II.

Standardisation of the extract RJM/0862/P08/A003 was carried out on the basis of 862-II by HPLC using following operating conditions :

| Column : | RP-18e (E-Merck, 5μm, 4.6 x 250 mm), at |
|---|---|
| Mobile Phase : | Acetonitrile (B) : 1.5% acetic acid in water (A) |
| Flow rate : | 1ml / min. |
| λmax : | 335 nm |

The compound was established as Verbascoside on the basis of NMR ($^1$H and $^{13}$C) and FABMS data.

The %age of 862-II in the extract RJM/0862/P08/A003 was found to be 0.22. 862-II, amorphous powder, mp 145-146°C,$[\alpha]^{21}$-85.6 [C 0.5% MeOH], MS: FABMS, $[M+Na]^+$ m/z 647 was found to be a known Phenyl propanoid i.e., Verbascoside (Acteoside); [β-(3',4'-dihydroxyphenyl) ethyl -O-α-L-rhamnopyranosyl (1→3)-β-D-(4-O-caffeoyl)-glucopyranoside]. Structure was finally confirmed by $^1$H and $^{13}$CNMR data similar to that reported in literature (Andary C., Wylde, R. Laffite C., Privat G. and Wintemitz F. ; Laboratie de Botanique et Cryptogamie, Faculte de Pharmacie. 34000 Montpellier. France; Phytochemistry, 21 (5), 1123-1127 (1982).

STUDY DESIGN

| 1. Animals | : Albino rats (Wistar, 150-180 g) either sex |
|---|---|
| | Albino mice (Swiss, 25-30 g) either sex |
| 2. Hepatotoxin | : Carbon tetrachloride (CCl$_4$) |
| 3. Study | : Prophylactic |
| 4. Treatment Schedule : | 48h, 24h, 02h, before and 06h after toxin; blood & liver samples collection 18 h after last treatment of test material / reference standard. |
| 5. Doses: | |
| Acteoside : | 0.625, 1.25, 2.5 & 5.0 mg/ kg, p.o. |
| Silymarin : | 50 mg/ kg, p.o. |
| Glycyrrhizin : | 100 mg/kg, p.o. |
| CCl$_4$ : | 1 ml/kg p.o. in liquid paraffin (1:1) |
| 6. Parameters | |
| Serum | |
| Bilirubin: | Jendrassik Method by using Kit supplied by Clonital, 24030-Carvico (BG)-Italy. |
| Triglycerides: | Enzymatic GFO-POD Method by the Kit supplied by Accurex BiomedicalPvt. Ltd., Thane |
| Albumin: | Colorimetric B.C.G. Method by the Kit supplied by Accurex Biomedical Pvt. Ltd., Thane |
| Transaminases | (ALT, & AST): Pyruvate formed by transamination reaction was determined spectrophotometrically after reaction with 2,4-dinitrophenylhydrazine (Reitman and Frankel, 1957). |
| ALP: | *p*-nitrophenol formed in alkaline medium was measured spectrophotometrically using p-nitrophenyl phosphate as substrate (Waler and Schutt, 1974). |
| Liver homogenate: | |
| GSH: | It was determined after deproteination by reaction with DTNB (Ellman 1959 as modified by David 1987). |
| Lipid peroxidation | :Thiobarbituric acid reacting substances were determined spectrophotometrically at 535 nm. By the method of Buege and Aust (1978). |

Hepatoprotective activity:

Hepatoprotective activity (H) was calculated by the following equation :

$$H = [ 1 - ( TC - V / VC - V ) ] \times 100$$

Where TC, VC, and V are drug + toxin, vehicle + toxin and vehicle treated groups of animals respectively.

**Table-1: Hepatoprotective potential of Acteoside against CCl$_4$ induced hepatic injury in Rodents Prophylactic).**

| Treatment | Dose mg/ kg | Serum parameter[a] | | | | | | Hepatic parameters | |
|---|---|---|---|---|---|---|---|---|---|
| | | GPT (Units) | GOT (Units) | ALP[b] | Bilirubin (mg %) | TG (mg %) | Albumin (g %) | LP[c] | GSH[d] |
| Vehicle | - | 90.76K ± 8.49 | 99.26±7.62 | 31.72±1.63 | 0.27±0.02 | 30.14±2.58 | 4.16±0.17 | 30.90±1.62 | 8.31±0278 |
| Veh+ CCl$_4$ | - | 1513.17±94.21 | 1006.20±33.43 | 76.40±3.68 | 0.76±0.02 | 90-85±4.12 | 2.76±0.11 | 84.35±3.97 | 3.25±0.17 |
| Acteoside only (Per-se) | 5 | 98.91±5,30 | 94.71±5.29 | 31.87±2.15 | 0.27±0.02 | 31.10±2.39 | 4.66±0.18 | 30.52±1.38 | 8.33±0.33 |
| Acteoside + CCl$_4$ | 0.625 | 852.27±47.93 (39.44) | 673.82±56.28 (36.64) | 66.80±3.53 (21.48) | 0.70±0.03 (12.24) | 80.15±3.53 (17.63) | 3.06±0.07 (21.43) | 61.08±3.39 (43.53) | 4.66±0.28 (27.86) |
| Acteoside + CCl$_4$ | 1.25 | 787.56±57.49 (51.01) | 503.42±48.95 (55.43) | 59.10±3.09 (38.72) | 0.59±0.03 (34.69) | 74.38±3.78 (27.13) | 3.25±0.14 (35.00) | 52.61±2.25 (59.38) | 5.93±0.32 (52.96) |
| Acteoside + CCl$_4$ | 2.5 | 662.62±39.80 (59.79) | 471.81±26.40 (58.92) | 51.20±1.42 (56.40) | 0.50±0.03 (53.06) | 59.85±1.88 (51.06) | 3.55±0.22 (56.43) | 45.46±2.79 (72.76) | 6.72±0.28 (68.57) |
| Acteoside + CCl$_4$ | 5.0 | 573.10±28.25 (66.08) | 366.89±26.97 (70.49) | 46.37±1.89 (67.21) | 0.42±0.02 (69.38) | 49.31±1.47 (68.42) | 3.91±0.12 (32.14) | 44.22±1.66 (75.07) | 7.02±0.46 (74.50) |
| Silymarin + CCl$_4$ | 50 | 665.48±50.08 (59.59) | 506.88±31.82 (55.06) | 52.47±2.43 (53.56) | 0.48±0.02 (57.14) | 58.94±3.35 (52.56) | 3.55±0.22 (53.57) | 55.23±4.66 (54.48) | 6.28±0.32 (59.88) |
| Glycyrrhizin +CCl$_4$ | 100 | 746.87 ± 49.77 (53.87) | 494.27 ± 36.28 (56.44) | 55.63 ± 3.52 (46.48) | 0.55 ± 0.04 (42.85) | 65.31 ± 3.69 (42.07) | 3.30 ± 0.12 (38.57) | 54.37 ± 4.32 (56.08) | 5.96 ±0.26 (53.55) |

a : Values represent mean ± SE of six animals in each group; Units : Each unit is μ mole pyruvate/min /L; b : μ mole of p-nitrophenol formed /min/L
c: lipid peroxidation (n mole MDA/g liver); d: glutathione (μ mole GSH/g liver).

Ŏ: Within parenthesis % protection

Ŏ: There is no effect on BP and Respiration in the doses of 30μg, 100μg, 300μg and 1 mg I.V. in rat as there is no change in base line.

Ŏ: There is no change in the base line against the Histamine, 5-HT and Acetylcholine induced contraction on the isolated rat colon.

EP 1 617 853 B1

**Table- 2: Significance analysis between the doses of acteoside (1.25, 2.5 and 5.0 mg/kg, p.o. at Df. 10 (Student *t* test)**

| Serum parameter[a] | | | | | | | | Hepatic parameters | |
|---|---|---|---|---|---|---|---|---|---|
| Dose: Dose | Student *t* test analysis | GPT (Units) | GOT (Units) | ALP[b] | Bilirubin (mg %) | TG (mg %) | Albumin (g %) | LP[c] | GSH[d] |
| 1.25 : 2.5 (a : b) | *t* value *P* value* | 1.78 < 0.20[NS] | 0.56 <0.20[NS] | 2.32 <0.05 | 2.12 < 0.10[NS] | 3.44 < 0.01 | 1.44 <0.10[NS] | 1.99 <0.10[NS] | 1.85 <0.10[NS] |
| 1.25 : 5.0 (a : c) | *t* value *P* value* | 3.34 <0.01 | 2.44 < 0.05 | 3.51 <0.01 | 4.71 < 0.001 | 6.18 < 0.001 | 3.58 < 0.01 | 3.00 < 0.01 | 1.94 < 0.10[NS] |
| 2.5 : 5.0 (b : c) | *t* value *P* value* | 1.83 <0.10[NS] | 2.78 <0.02 | 2.04 <0.01[NS] | 2.22 < 0.05 | 4.41 < 0.01 | 1.43 <0.10[NS] | 0.38 <0.10[NS] | 0.55 <0.10[NS] |

a : Values represent mean ± SE of six animals in each group;
Units: Each unit is µ mole pyruvate/min /L;
b : µ mole of p-nitrophenol formed /min/L
c: lipid peroxidation (n mole MDA/g liver);
d: glutathione (p mole GSH/g liver).
* $p < 0.05$, ** $p < 0.01$, $P < 0.001$, $p > 0.05$, [not significant] *Student t* test between the respective does.

**Claims**

1. A process of preparing Acteoside, said process comprising extracting acteoside from plant *Colerbrookea oppositifolia.*

2. A process of isolation of pure Acteoside of high hepatoprotection from plant *Colerbrookea oppositifolia,* said process comprising steps of:

   a. drying aerial parts of the plant,
   b. grinding the dried parts into powder,
   c. percolating the powder with water or ethanol for 3-4 times to obtain an extract,
   d. filtering the extract for clearing suspended particles to obtain supernatant,
   e. drying the supernatant at about 45 to 55°C to obtain a residue,
   f. fractionating the residue with chloroform, ethyl acetate, and butanol successively,
   g. subjecting butanol fraction to adsorption chromatography of $SiO_2$ after adding methanol to the fraction,
   h. charging the adsorbed fraction to glass column,
   i. eluting the column with solvents of increasing polarity of methanol :

   chloroform to obtain further fractions and repeating the process one more time,

   j. subjecting the fractions to column chromatography to obtain fractions,
   k. concentrating the fractions under reduced pressure to obtain acteoside as residue.

3. A process as claimed in claim 1 or 2, wherein the extracts are aqueous and alcoholic extracts.

4. A process as claimed in claim 1 or 2, wherein acteoside is about 1.0% (wt.) of the total extract.

5. An extract from colerbrookea oppositifolia comprising Acteoside obtainable by the process according to claim or 2.

6. Use of an extract according to claim 5 for the manufacture of a medicament for use in a method of protecting a subject against hepatotoxicity.

**7.** Use of an extract according to claim 5 for the manufacture of a medicament for use in a method of protecting a subject against hepatotoxicity, said wherein the extract is to be administered to the subject with acteoside in a dosage ranging between 0.5 and 10.0 mg/kg bodyweight.

**8.** Use of an extract according to claim 5 for the manufacture of a medicament for use in method of reducing in a subject abnormally elevated levels of serum glutamine transferase (GPT), serum glutamine transferase (GOT), serum alkaline phosphatase (ALP), Bilirubin, serum triglycerides (TG), lipid peroxidase (LP), albumin and/or reduced-glutathione, wherein the extract is to be administered to the subject with acteoside in an amount ranging between 0.5 and 10.0 mg/kg bodyweight.

**9.** Use according to any one of claims 6 to 8, wherein acteoside is to be administered through P.O. routes.

**Patentansprüche**

**1.** Verfahren zur Herstellung von Acteosid, wobei das Verfahren Extrahieren von Acteosid aus der Pflanze *Colerbrookea oppositifolia* umfasst.

**2.** Verfahren zur Isolierung von reinem Acteosid mit hoher Hepatoprotektion aus der Pflanze *Colerbrookea oppositifolia,* wobei das Verfahren folgende Schritte umfasst:

    a. Trocknen von oberirdischen Teilen der Pflanze,
    b. Mahlen der getrockneten Teile zu Pulver,
    c. drei- bis viermaliges Durchsickern lassen von Wasser oder Ethanol durch das Pulver, um einen Extrakt zu erhalten,
    d. Filtrieren des Extrakts zum Entfernen von suspendierten Teilchen, um den Überstand zu erhalten,
    e. Trocknen des Überstands bei etwa 45 °C bis 55 °C, um einen Rückstand zu erhalten,
    f. aufeinanderfolgendes Fraktionieren des Rückstands mit Chloroform, Ethylacetat und Butanol,
    g. Unterziehen der Butanolfraktion einer Adsorptionschromatographie mit $SiO_2$ nach Zugeben von Methanol zur Fraktion,
    h. Beladen der adsorbierten Fraktion auf eine Glassäule,
    i. Eluieren der Säule mit Lösungsmitteln steigender Polarität aus Methanol:Chloroform, um weitere Fraktionen zu erhalten, und Wiederholen des Verfahrens ein weiteres Mal,
    j. Unterziehen der Fraktionen einer Säulenchromatographie, um Fraktionen zu erhalten,
    k. Konzentrieren der Fraktionen unter vermindertem Druck, um Acteosid als Rückstand zu erhalten.

**3.** Verfahren gemäß Anspruch 1 oder 2, wobei die Extrakte wässrige und alkoholische Extrakte sind.

**4.** Verfahren gemäß Anspruch 1 oder 2, wobei Acteosid etwa 1,0 Gew.-% des gesamten Extrakts ausmacht.

**5.** Extrakt aus *Colerbrookea oppositifolia,* umfassend Acteosid, erhältlich durch das Verfahren gemäß Anspruch 1 oder 2.

**6.** Verwendung eines Extrakts gemäß Anspruch 5 zur Herstellung eines Medikaments zur Verwendung in einem Verfahren zum Schutz eines Patienten gegen Hepatotoxizität.

**7.** Verwendung eines Extrakts gemäß Anspruch 5 zur Herstellung eines Medikaments zur Verwendung in einem Verfahren zum Schutz eines Patienten gegen Hepatotoxizität, wobei der Extrakt dem Patienten mit Acteosid in einer Dosierung im Bereich von 0,5 bis 10,0 mg/kg Körpergewicht zu verabreichen ist.

**8.** Verwendung eines Extrakts gemäß Anspruch 5 zur Herstellung eines Medikaments zur Verwendung in einem Verfahren zum Reduzieren abnormal erhöhter Spiegel von Serumglutamintransferase (GPT), Serumglutamintransferase (GOT), Serum alkalische Phosphatase (ALP), Bilirubin, Serumtriglyceriden (TG), Lipidperoxidase (LP), Albumin und/oder reduziertem Glutathion in einem Patienten, wobei der Extrakt dem Patienten mit Acteosid in einer Menge im Bereich von 0,5 bis 10,0 mg/kg Körpergewicht zu verabreichen ist.

**9.** Verwendung gemäß einem der Ansprüche 6 bis 8, wobei Acteosid auf oralem Weg zu verabreichen ist.

**Revendications**

1. Procédé de préparation d'Actéoside, ledit procédé comprenant l'extraction d'Actéoside de la plante *Colerbrookea oppositifolia.*

2. Procédé d'isolement d'Actéoside pur à hépatoprotection élevée à partir de la plante *Colerbrookea oppositifolia,* ledit procédé comprenant les étapes consistant à :

   a. sécher les parties aériennes de la plante,
   b. broyer les parties séchées sous forme d'une poudre,
   c. effectuer 3-4 fois une percolation de la poudre avec de l'eau ou de l'éthanol pour obtenir un extrait,
   d. filtrer l'extrait pour éliminer les particules en suspension afin d'obtenir un surnageant,
   e. sécher le surnageant à environ 45 à 55 °C pour obtenir un résidu,
   f. fractionner le résidu avec du chloroforme, de l'acétate d'éthyle et du butanol, successivement,
   g. soumettre la fraction butanolique à une chromatographie d'adsorption de $SiO_2$ après avoir ajouté du méthanol à la fraction,
   h. charger la fraction adsorbée dans une colonne de verre,
   i. éluer la colonne avec des solvants de polarité croissante de méthanol: chloroforme pour obtenir d'autres fractions et répéter le procédé une fois de plus,
   j. soumettre les fractions à une chromatographie sur colonne pour obtenir des fractions,
   k. concentrer les fractions sous pression réduite pour obtenir de l'Actéoside sous forme d'un résidu.

3. Procédé selon la revendication 1 ou 2, dans lequel les extraits sont des extraits aqueux et alcooliques.

4. Procédé selon la revendication 1 ou 2, dans lequel l'Actéoside représente environ 1,0 % (en poids) de l'extrait total.

5. Extrait de *Colerbrookea oppositifolia* comprenant de l'Actéoside que l'on peut obtenir par le procédé selon la revendication 1 ou 2.

6. Utilisation d'un extrait selon la revendication 5 pour la fabrication d'un médicament à utiliser dans un procédé de protection d'un patient contre une hépatotoxicité.

7. Utilisation d'un extrait selon la revendication 5 pour la fabrication d'un médicament à utiliser dans un procédé de protection d'un patient contre une hépatotoxicité, dans laquelle l'extrait doit être administré au patient avec de l'Actéoside en une posologie située dans l'intervalle de 0,5 à 10,0 mg/kg de poids corporel.

8. Utilisation d'un extrait selon la revendication 5 pour la fabrication d'un médicament à utiliser dans un procédé de réduction, chez un patient, de taux anormalement élevés de glutamine-transférase sérique (GPT), de glutamine-transférase sérique (GOT), de phosphatase alcaline sérique (ALP), de bilirubine, de triglycérides sériques (TG), de peroxydase lipidique (LP), d'albumine et/ou de glutathion réduit, dans laquelle l'extrait doit être administré au patient avec de l'Actéoside en une quantité située dans l'intervalle de 0,5 à 10,0 mg/kg de poids corporel.

9. Utilisation selon l'une quelconque des revendications 6 à 8, dans laquelle l'Actéoside doit être administré par des voies p.o.

1: 335 nm, 8 nm
U62-II ( lst )
KAS 2-12-02-Rep7
Retention Time

Acetoside

**1: 335 nm, 8 nm**

| Pk # | Retention Time | Area | Area % | Height | Height % |
|---|---|---|---|---|---|
| 1 | 43.979 | 39514 | 0.020 | 1079 | 0.051 |
| 2 | 47.083 | 193818435 | 99.928 | 2128832 | 99.848 |
| 3 | 53.579 | 100792 | 0.052 | 2168 | 0.102 |
| Totals | | 193958741 | 100.000 | 2132079 | 100.000 |

Fig. 1

EP 1 617 853 B1

## Comparative hepatoprotective activity (%) of acteoside, silymarin and glycyrrhizin

Legend: ALT  AST  ALP  Bilirubin  TG  Albumin  LP  GSH

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **QUANBO XING ; KOJI HASE et al.** *Planta Medica,* 1998, vol. 64, 120-125
- **PETER J. HOUGHTON ; HIROSHI HIKINO.** *Planta Medica,* 1989, vol. 55, 123-126
- **S. AZIZ AHMED ; S.A. SIDDIQUI ; ASIF ZAMAN.** *Indian J. Chemistry,* 1974, vol. 12, 1327-28
- **S.A. PATWARDHAN ; A.S.GUPTA.** *Indian J. Chemistry,* 1981, vol. 20B, 627
- **FAN YANK ; XING-LI ; HAN-QING WANG ; CHONG-REN YANG.** *Phytochemistry,* 1996, vol. 42, 867-69
- **R.S. GUPTA ; R.J. YADAV ; V.P. DIXIT ; M.P. DOBHAL.** *Fitoterapia,* 2001, vol. 72, 236-45
- **PETER J. HOUGHTON ; HIROSHI HIKINO.** *Planta Medica,* 1959, vol. 55, 123-126
- *Phytochemistry,* 1982, vol. 21, 1123-1127
- **ANDARY C. ; WYLDE, R. ; LAFFITE C. ; PRIVAT G. ; WINTEMITZ F.** *Phytochemistry,* 1982, vol. 21 (5), 1123-1127